# EUROPÄISCHE PATENTSCHRIFT

(11) **EP 4 294 054 B1**
(45) Veröffentlichungstag und Bekanntmachung des Hinweises auf die Patenterteilung: **31.12.2025**
(21) Anmeldenummer: 23173085.4
(22) Anmeldetag: 12.05.2023
(51) Int. Cl.: H04R 25/02, H04R 1/10, A61B 8/02, A61B 5/00, A61B 5/024

(54) **VERFAHREN ZUR PULSMESSUNG EINER PERSON MITTELS EINES HÖRSYSTEMS**
METHOD FOR PULSE MEASUREMENT OF A PERSON BY MEANS OF A HEARING SYSTEM
PROCÉDÉ DE MESURE DE POULS D'UNE PERSONNE AU MOYEN D'UN SYSTÈME AUDITIF

(30) Priorität: 14.06.2022 DE 102022206012
(43) Veröffentlichungstag der Anmeldung: 20.12.2023
(73) Patentinhaber: Sivantos Pte. Ltd., Singapore 539775 (SG)
(72) Erfinder: GÖKAY, Umut, 91058 Erlangen (DE)
(74) Vertreter: FDST Patentanwälte

(56) Entgegenhaltungen:
- WO-A1-2018/205013
- WO-A2-2009/069037
- CN-A- 104 244 127
- US-A1- 2019 022 349
- US-A1- 2021 360 354

## Beschreibung

Die Erfindung betrifft ein Verfahren zur Pulsmessung einer Person mittels eines Hörsystems. Die Erfindung betrifft weiter ein Hörsystem, welches für ein solches Verfahren eingerichtet ist.

Ein Hörinstrument ist allgemein als ein Gerät aufzufassen, welches dazu eingerichtet ist, aus einem elektrischen Audiosignal (welches auch durch ein internes Verarbeitungssignal des Gerätes gegeben sein kann) durch einen elektroakustischen Ausgangswandler (z.B. einen Lautsprecher) einen Ausgangsschall zu erzeugen, welcher dem Gehör eines Trägers zugeführt wird. Das Hörinstrument kann dabei z.B. als Kopfhörer, insbesondere als Earplug-artiger Kopfhörer ausgestaltet sein. Das Hörinstrument kann aber auch als ein Hörgerät "im engeren Sinn" gegeben sein, welches dazu vorgesehen und eingerichtet ist, eine Hörschwäche des Trägers zu korrigieren, indem ein Umgebungsschall durch mindestens einen elektroakustischen Eingangswandler (z.B. ein Mikrofon) in ein Eingangssignal umgewandelt wird, welches gemäß den audiologischen Anforderungen des Trägers verarbeitet und dabei insbesondere frequenzbandspezifisch verstärkt wird. Ein aus der Verarbeitung resultierendes Audiosignal wird dann durch einen elektroakustischen Ausgangswandler des Hörinstruments in einen Ausgangsschall umgewandelt.

Hörsysteme, also Systeme mit wenigstens einem Hörinstrument, werden zunehmend mit der Funktion ausgestattet, eine Herzfrequenz eines Trägers des Hörsystems zu messen. Dies ist einerseits der Fall bei Kopfhörern mit einer Fitness-Funktion, welche der Träger während einer sportlichen Betätigung trägt. Die Kopfhörer können dabei noch weitere physiologische Parameter überwachen, oder das Hörsystem kann zusätzlich auch eine Smartwatch o.ä. umfassen, mittels derer weitere solche Parameter überwacht werden können. Überdies kann der Träger bei der genannten sportlichen Betätigung zur Ablenkung z.B. Musik hören. Andererseits können auch Hörgeräte im engeren Sinn solche Funktionen aufweisen. In diesem Fall wird dabei insbesondere dem Umstand Rechnung getragen, dass Hörgeräte nicht selten von älteren Personen getragen werden, für welche eine Überwachung des Gesundheitszustands gewünscht oder gar indiziert ist.

Eine Messung der Herzfrequenz kann durch ein Hörinstrument z.B. mittels einer Photoplethysmographie (PPG) erfolgen. Ein PPG-Sensor emittiert dabei mittels einer oder mehrerer LEDs Licht verschiedener Wellenlänge ins Gewebe, und erfasst anschließend im Wesentlichen das vom Gewebe transmittierte und/oder reflektierte Licht. Dieses Licht, welches teils durch Blutgefäße unter der Hautoberfläche propagiert, ist dabei den periodischen Variationen des Pulsschlags unterworfen, sodass anhand dieser Variationen die Herzfrequenz ermittelt werden kann.

Ein Nachteil ist jedoch, dass die hier beschriebene Pulsmessung mittels PPG bei einer dauerhaften Anwendung einen hohen Energieverbrauch für die eingesetzten LEDs zur Folge hat. Gerade bei Hörinstrumenten ist jedoch die für den Betrieb verfügbare Batterieleistung oftmals sehr beschränkt.

Die WO 2018 / 205 013 A1 nennt ein Verfahren und ein zugehöriges System zur Bestimmung der Herz- und/oder Atemfrequenz. Das System umfasst ein Gehäuse, die dazu ausgelegt ist, einen Gehörgang zu verschließen, ein In-Ohr-Mikrofon, das sich innerhalb des Gehäuses befindet, um ein Audiosignal innerhalb des Gehörgangs zu erfassen, und einen Prozessor, der mit dem In-Ohr-Mikrofon verbunden ist, wobei der Prozessor dazu eingerichtet ist, das Audiosignal dahingehend zu analysieren, um mindestens eine Herzschlag- und/oder Atemfrequenzmessung, selbst wenn der Träger des Systems einem hohen Pegel von Umgebungsgeräuschen ausgesetzt ist. Das System und das Verfahren umfassen auch die Verwendung eines adaptiven digitalen Filters, um das restliche Umgebungsrauschen aus dem vom In-Ohr-Mikrofon aufgenommenen Audiosignal zu entfernen.

In der US 2019 / 0 022 349 A1 ist ein Ohrhörer mit einem Lautsprecher, einem Mikrofon, einem den Lautsprecher und das Mikrofon tragenden Gehäuse, und einem Ohrstöpsel genannt. Der Ohrstöpsel umgibt das Gehäuse und ist dazu ausgelegt, dass er sowohl den Lautsprecher als auch das Mikrofon akustisch an einen Gehörgang eines Benutzers koppelt und den Eingang zum Gehörgang des Benutzers akustisch verschließt. Ein Prozessor empfängt Eingangsaudiosignale vom Mikrofon, detektiert Spitzen mit einer Frequenz von etwa 1 Hz in den Eingangsaudiosignalen, berechnet auf der Grundlage der detektierten Spitzen eine momentane Herzfrequenz, misst eine Frequenz einer Oszillation innerhalb der momentanen Herzfrequenz und berechnet auf der Grundlage der Frequenz der Oszillation eine Atmungsrate.

Die CN 104 244 127 A offenbart ein Verfahren zur Erfassung der Herzfrequenz mittels eines Ohrhörers, welcher ein Mikrofon und einen Beschleunigungssensor umfasst. Für das Verfahren wird eine adaptive Filterverarbeitung an einem von dem Beschleunigungssensor erfassten Signal durchgeführt, so dass ein Schätzsignal eines Signals erzeugt wird, welches durch die Körperbewegung eines Trägers in einem vom Mikrofon erzeugten Mikrofonsignal erhalten wird; das Schätzsignal wird vom Mikrofonsignal subtrahiert, und ein auf die Herzfrequenz bezogenes Signal wird erhalten, auf welchem die Herzfrequenzerfassung durchgeführt wird.

In der US 2021 / 0 360 354 A1 wird eine Hörhilfe zum Tragen an oder in einem Ohr eines Benutzers offenbart, welche einen Herzschlagdetektor, der ein Pulssteuersignal liefert, und einen Prozessor umfasst. Der Prozessor ist dazu eingerichtet, in Abhängigkeit vom Pulssteuersignal schätzt, ob sich das Hörgerät am linken oder am rechten Ohr des Benutzers befindet.

Aus der WO 2009 / 069 037 A2 ist ein Herzmonitor mit einem elektroakustischen Eingangswandler, der mit einer Steuereinheit verbunden ist, bekannt. Der Eingangswandler wird im Ohr einer Person positioniert und steht in akustischer Verbindung mit dem Trommelfell. Die Signale des Eingangswandler werden verarbeitet, um das Vorhandensein eines pulsierenden Blutflusses festzustellen. Der Herzmonitor kann in ein tragbares Medienwiedergabegerät eingebaut werden, welches zwischen einem Wiedergabe- und einem Überwachungsmodus wechselt oder auch beide Modi gleichzeitig ausführen kann. Der Herzmonitor kann mit einem Defibrillator verbunden werden, um das Vorhandensein eines Blutflusses zu erkennen und bei der Entscheidung über die Schockabgabe zu berücksichtigen.

Es ist daher Aufgabe der Erfindung, ein Verfahren für ein Hörsystem mit wenigstens einem Hörinstrument bereitzustellen, mittels dessen sich möglichst energieeffizient und zuverlässig eine Pulsmessung eines Trägers des Hörsystems durchführen lässt. Es ist weiter Aufgabe der Erfindung, ein entsprechendes Hörsystem bereitzustellen.

Die erstgenannte Aufgabe wird erfindungsgemäß gelöst durch ein Verfahren zur Pulsmessung einer Person mittels eines Hörsystems, wobei das Hörsystem wenigstens ein erstes Hörinstrument mit einem elektroakustischen ersten Eingangswandler umfasst, wobei durch den ersten Eingangswandler ein erstes Körperschallsignal an einem Ohr der Person aufgenommen wird, und hierdurch ein erstes Eingangssignal erzeugt wird.

Verfahrensgemäß ist hierbei vorgesehen, dass anhand eines Amplitudenverlaufes des ersten Eingangssignals eine Pulsfrequenz ermittelt wird. Vorteilhafte und teils für sich gesehen erfinderische Ausgestaltungen sind Gegenstand der Unteransprüche und der nachfolgenden Beschreibung.

Erfindungsgemäß ist dabei zudem vorgesehen, dass in einem Spezialmodus, durch einen ersten Sensor des Hörsystems und/oder einen zweiten Eingangswandler des Hörsystems ein Hilfssignal erzeugt wird, anhand des Hilfssignals eine erste Information über einen Pulsschlag der Person bezogen wird, und daraufhin anhand der ersten Information und anhand des ersten Eingangssignals die Pulsfrequenz ermittelt wird.

Insbesondere kann die Pulsfrequenz auch anhand einer ersten Information ermittelt werden, wobei die erste Information wenigstens zwei Extrema und/oder wenigstens zwei steigende oder fallende Flanken von einer Einhüllenden des ersten Eingangssignals und/oder vor einer Betragsfunktion des ersten Eingangssignals umfasst. Die erste Information kann somit also anhand des Hilfssignals gewonnen werden, welches durch den ersten Sensor und/oder einen zweiten Eingangswandler erzeugt wird, oder auch aus dem Verlauf des ersten Eingangssignals selbst.

Die zweitgenannte Aufgabe wird erfindungsgemäß gelöst durch ein Hörsystem, welches wenigstens ein erstes Hörinstrument mit einem elektroakustischen ersten Eingangswandler, und eine Steuereinheit sowie vorzugsweise weiter einen ersten Sensor und/oder einen zweiten Eingangswandler umfasst, wobei das Hörsystem dazu eingerichtet ist, das vorbeschriebene Verfahren zur Pulsmessung einer Person durchzuführen, wenn wenigstens das erste Hörinstrument, insbesondere bestimmungsgemäß, an einem Ohr der Person getragen wird. Insbesondere ist hierbei der erste Eingangswandler dazu eingerichtet, ein erstes Körperschallsignal an einem Ohr der Person aufzunehmen, und hierdurch ein erstes Eingangssignal zu erzeugen, und wobei die Steuereinheit dazu eingerichtet ist, anhand eines Amplitudenverlaufes des ersten Eingangssignals eine Pulsfrequenz zu ermitteln. Insbesondere kann dabei der erste Sensor des Hörsystems bzw. der zweite Eingangswandler des Hörsystems dazu eingerichtet sein, ein Hilfssignal zu erzeugen, welches Aufschluss über den Pulsschlag der Person liefert, sodass wenigstens zeitweise anhand des Hilfssignals eine erste Information über den Pulsschlag der Person bezogen werden kann, und daraufhin die Steuereinheit die Pulsfrequenz auch anhand der ersten Information (und anhand des ersten Eingangssignals) ermittelt.

Das erfindungsgemäße Hörsystem teilt die Vorzüge des erfindungsgemäßen Verfahrens, und ist insbesondere zur Durchführung des Verfahrens durch eine entsprechende Ausgestaltung und Konfigurierung eingerichtet. Die für das Verfahren und für seine Weiterbildungen angegebenen Vorteile können dabei sinngemäß auf das Hörsystem übertragen werden.

Unter einem Hörinstrument ist generell jedwedes Gerät umfasst, welches dazu vorgesehen und eingerichtet ist, aus einem elektrischen Ausgangssignal mittels eines elektroakustischen Ausgangswandlers einen entsprechenden Ausgangsschall zu erzeugen, und diesen einem Gehör eines Benutzers zuzuführen. Als ein solcher Ausgangswandler kann hierbei insbesondere ein Lautsprecher verwendet werden, jedoch können bspw. auch thermoakustische Wandler verwendet werden. Ein Hörinstrument kann dabei einerseits lediglich auf die Erzeugung des Ausgangsschalls anhand von Audiodaten ausgelegt sein, also beispielsweise in der Form eines drahtlosen, insbesondere Ohrstöpsel-förmigen Kopfhörers. **In** diesem Fall wird ein Ausgangsschall anhand von Audiodaten erzeugt, welche beispielsweise durch Musik gegeben sein können, und welche vorab gespeichert wurden, oder auch über eine entsprechende Antenne zum Hörinstrument übertragen werden (per Stream).

Ein Hörinstrument kann aber durch ein Hörgerät ("im engeren Sinn") gegeben sein, welches dazu eingerichtet ist, eine Hörschwäche eines Benutzers zu korrigieren bzw. wenigstens teilweise auszugleichen, indem beispielsweise mittels wenigstens eines elektroakustischen Eingangswandlers wie z.B. einem Mikrofon (oder mehreren Mikrofonen) ein Umgebungsschall in ein entsprechendes elektrisches Eingangssignal umgewandelt wird, welches im Hörgerät gemäß der audiologischen Anforderungen des Benutzers verarbeitet und dabei insbesondere frequenzbandweise verstärkt wird, sodass das verarbeitete Eingangssignal über den elektroakustischen Ausgangswandler dem Gehör des Benutzers als Ausgangsschall zugeführt wird.

Unter einem elektroakustischen Eingangswandler ist hierbei insbesondere ein solcher Wandler umfasst, welcher dazu eingerichtet ist, aus dem Umgebungsschall ein entsprechendes elektrisches Signal zu erzeugen. Insbesondere kann bei der Erzeugung des ersten bzw. zweiten Eingangssignals durch den jeweiligen Eingangswandler auch eine Vorverarbeitung erfolgen, z.B. in Form einer linearen Vorverstärkung und/oder einer A/D-Konvertierung. Das entsprechend erzeugte Eingangssignal ist dabei insbesondere durch ein elektrisches Signal gegeben, dessen Strom- und/oder Spannungsschwankungen im Wesentlichen die Schalldruck-Schwankungen der Luft repräsentieren.

Das durch den ersten Eingangswandler am Ohr der Person aufgezeichnete erste Körperschallsignal beinhaltet bevorzugt einen Schallanteil, welcher einem Pulsschlag entspricht, bzw. einem solchen zuzuordnen ist. Das erste Hörinstrument wird dabei von der Person in oder an einem Ohr getragen, sodass das erste Körperschallsignal durch den ersten Eingangswandler des ersten Hörinstruments im bzw. am Ohr aufgezeichnet wird. Entsprechend beinhaltet das erste Körperschallsignal Schallanteile eines Pulsschlags im Bereich des betreffenden Ohres, vorzugsweise im Gehörgang. Hierfür ist bevorzugt der erste Eingangswandler derart im bzw. am ersten Hörinstrument angeordnet, dass er bei einem bestimmungsgemäßem Tragen des ersten Hörinstrument in den Gehörgang gerichtet ist, welcher durch das erste Hörinstrument wenigstens teilweise verschlossen wird. Der erste Eingangswandler nimmt dabei bevorzugt ein erstes Körperschallsignal des okkludierten Gehörgangs mit auf, d.h., der Pulsschlag der den Gehörgang umgebenden Blutgefäße erzeugt im (wenigstens teilweise) verschlossenen Gehörgang ein pulsierendes Geräusch, welches infolge der Okklusion nicht (vollständig) entweichen kann, und somit als entsprechender Schallanteil in das erste Körperschallsignal eingeht und hierdurch auch in das erste Eingangssignal eingeht.

Als erster Sensor des Hörsystems ist insbesondere ein PPG-Sensor umfasst, aber auch ein an einer Hals- oder Kopfschlagader angeordneter Beschleunigungssensor, oder ein EKG-Sensor mit entsprechenden Elektroden, welche im Betrieb des Hörsystems an geeigneter Stelle am Körper der Person angeordnet sind. **In** erstgenannten Fall des PPG-Sensors ist dieser bevorzugt im ersten Hörinstrument angeordnet. Das Hörsystem kann jedoch auch ein oder mehrere weitere Geräte wie z.B. eine Smartwatch o.ä. umfassen, welche mit dem ersten Hörinstrument datentechnisch verbindbar sind. Der erste Sensor kann dann in einem solchen weiteren Gerät angeordnet sein.

Der erste Sensor ist dabei bevorzugt dazu eingerichtet, als das Hilfssignal ein Sensorsignal zu erzeugen, anhand dessen sich die erste Information über den Pulsschlag der Person beziehen lässt. Insbesondere kann das besagte Sensorsignal (also das Hilfssignal) eine eigenständige Pulsmessung ermöglichen, oder zumindest einen Rückschluss über eine Pulsfrequenz und/oder ein Phase eines Pulsschlags erlauben.

Insbesondere kann als Hilfssignal jedoch auch ein zweites Eingangssignal eines elektroakustischen zweiten Eingangswandlers des Hörsystems erzeugt werden. In diesem Fall ist der zweite Eingangswandler insbesondere in einem zweiten Hörinstrument eines binauralen Hörsystems angeordnet. Die Erzeugung des zweiten Eingangssignals als Hilfssignal erfolgt dann in vergleichbarer Weise zum ersten Eingangssignal, wobei ein zweites Körperschallsignal aufgezeichnet wird.

Anhand der ersten Information, welche bevorzugt eine Referenz-Pulsfrequenz und/oder eine Phase eines Pulsschlags umfassen, kann nun im direkt ersten Eingangssignal eine Messung der Pulsfrequenz durchgeführt werden. Dies kann z.B. durch eine Korrelationsmessung oder etwa auch durch ein sog. "Minimum Tracking" der Signalamplitude erfolgen, wobei die erste Information zusätzlich als eine Referenz herangezogen wird.

Ist nämlich ein Signal-zu-Rausch-Verhältnis ("signal-to-noise-ratio", SNR) des Schallanteils des Pulsschlags im ersten Eingangssignal unvorteilhaft, so kann für das Ermitteln der ersten Pulsfrequenz zusätzlich auf die Referenz der ersten Information zurückgegriffen werden. Hierbei kann das Hilfssignal insbesondere nur dann herangezogen werden, wenn es tatsächlich erforderlich ist, da z.B. im ersten Eingangssignal ein ungünstiges SNR für eine Pulsmessung vorliegt.

Insbesondere kann dabei also auch eine eigenständige Pulsmessung anhand eines Hilfssignals aus einer PPG-Messung o.ä. durchgeführt werden. Die so ermittelte Pulsfrequenz als erste Information wird dann im weiteren Verlauf für das Ermitteln der Pulsfrequenz nur anhand aus den Signalanteilen des ersten Eingangssignals als Referenz verwendet (z.B. für eine Korrelationsmessung).

Erfindungsgemäß wird in einem Normalmodus die Pulsfrequenz anhand des ersten Eingangssignals ermittelt, insbesondere anhand des Amplitudenverlaufes und besonders bevorzugt ohne eine Erzeugung und/oder ohne eine Verwendung aktueller Werte des Hilfssignals, wobei im Fall eines Fehlers beim Ermitteln der Pulsfrequenz im Normalmodus in einen Spezialmodus gewechselt wird, und wobei im Spezialmodus anhand des Hilfssignals oder anhand einer Korrelationsmessung die erste Information über einen Pulsschlag der Person bezogen wird, und die Pulsfrequenz anhand der ersten Information (und ggf. anhand des ersten Eingangssignals) ermittelt wird. Dies bedeutet insbesondere, dass im Normalbetrieb des Hörsystems das für die Pulsmessung relevante Messsignal das erste Eingangssignal ist, welches die aktuelle Information über den Pulsschlag trägt. Die Messung der Pulsfrequenz wird dann nur am aktuellen ersten Eingangssignal durchgeführt, ggf. jedoch anhand von zusätzlichen Informationen, welche zu einem vorangegangenen Zeitpunkt anhand des Hilfssignals gewonnen wurden. Aus diesem Grund ist im Normalmodus eine Erfassung des Hilfssignals nicht erforderlich.

Erst im Fehlerfall, also insbesondere, wenn sich im Normalmodus die Pulsfrequenz nicht aus dem ersten Eingangssignal ermitteln lässt (etwa, da das SNR zu schlecht ist, oder weil infolge einer Änderung der körperlichen Aktivität der Person eine Referenz verloren gegangen ist), wird die erste Information aus dem Hilfssignal bezogen, und somit in den Spezialmodus gewechselt.

Bevorzugt wird dann nach einem erfolgreichen Ermitteln der Pulsfrequenz im Spezialmodus wieder in den Normalmodus gewechselt, also insbesondere ein Beziehen der ersten Information und vorzugsweise auch eine Erfassung des Hilfssignals ausgesetzt. Ist das Hilfssignal durch ein zweites Eingangssignal des Hörsystems gegeben, welches im laufenden Betrieb des Hörsystems kontinuierlich erzeugt wird (und insbesondere auch anderweitige Verwendung im Betrieb findet), so wird bei einer Rückkehr in den Normalmodus insbesondere eine Verwendung der aktuellen Werte des Hilfssignals für die Pulsmessung ausgesetzt. Dies erlaubt eine besonders energieeffiziente Messung der Pulsfrequenz, da die erhöhte Präzision des Hilfssignals nur dann verwendet wird (bzw. die erste Information nur dann bezogen wird), wenn es erforderlich ist, nämlich wenn eine Pulsmessung im Normalmodus zunächst scheitert, und somit in den Spezialmodus gewechselt wird. Der höhere Energieverbrauch im Spezialmodus infolge des ersten Sensors wird dann nur aufrechterhalten, bis die Pulsfrequenz wieder mithilfe der Referenz der ersten Information erfasst werden kann.

Vorzugsweise wird dabei nach dem Wechsel vom Spezialmodus in den Normalmodus die Pulsfrequenz anhand des ersten Eingangssignals und der im vorigen Spezialmodus bezogenen ersten Information ermittelt. Dies umfasst insbesondere, dass z.B. eine im Spezialmodus anhand eines Hilfssignals aus einer PPG-Messung ermittelte Pulsfrequenz nach einer Rückkehr in den Normalmodus als Referenz für eine Messung der Pulsfrequenz lediglich anhand der aktuellen Signalbeiträge des ersten Eingangssignals verwendet wird, und die PPG-Messung dabei im Normalmodus ausgesetzt wird.

Zweckmäßigerweise wird die Pulsfrequenz (wenigstens im Normalmodus) anhand einer Korrelationsmessung des ersten Eingangssignals ermittelt, wobei insbesondere anhand der ersten Information ein Parameter der Korrelationsmessung bestimmt wird.

Hierbei kann insbesondere die Korrelation des ersten Eingangssignals mit einer Testfunktion ermittelt werden, welcher eine vorgegebene Frequenz zugeordnet ist bzw. welche eine vorgegebene Frequenz aufweist. Die Testfunktion modelliert hierbei bevorzugt einen bestimmten Signalverlauf, insbesondere des ersten Eingangssignals, für einen Pulsschlag der vorgegebenen Frequenz. Die Testfunktion ist hierfür bevorzugt anhand der ersten Information auszuwählen aus einer Mehrzahl an möglichen Testfunktionen unterschiedlicher Frequenz und ggf. auch unterschiedlicher Signalverläufe. Als Parameter der Korrelationsmessung kann dann insbesondere die Frequenz der Testfunktion anhand der ersten Information vorgegeben werden. Insbesondere kann dabei für die besagte Mehrzahl an verschiedenen möglichen Testfunktionen mit unterschiedlicher Frequenz jeweils eine Korrelation des ersten Eingangssignals mit einer Testfunktion aus der Mehrzahl ermittelt werden, wobei die erste Testfunktion aus der Mehrzahl anhand eines Maximums der besagten Korrelationen ermittelt wird. Die erste Testfunktion ist somit insbesondere diejenige Testfunktion aus der besagten Mehrzahl an möglichen Testfunktionen, welche die höchste Korrelation mit dem ersten Eingangssignal aufweist.

Bevorzugt wird dabei im Spezialmodus anhand des Maximums der besagten Korrelationen als erste Information die erste Testfunktion aus der Mehrzahl an Testfunktionen und/oder die Frequenz der ersten Testfunktion ermittelt wird. Mit anderen Worten bedeutet dies insbesondere, dass im Spezialmodus die erste Information gegeben ist durch die erste Testfunktion selbst bzw. durch die Identifikation der ersten Testfunktion anhand ihrer Frequenz.

Günstigerweise wird die erste Information bezogen, wenn der Wert der Korrelationsmessung unter einen vorgegebenen Grenzwert fällt. Insbesondere kann dabei der Wert der Korrelationsmessung auch als Kriterium für einen Wechsel vom Normalmodus in den Spezialmodus herangezogen werden. Wird z.B. die Korrelationsmessung gebildet als eine Kreuzkorrelation des ersten Eingangssignal mit der vorgegebenen Testfunktion, so kann anhand des Maximums über das Argument der Kreuzkorrelation die Phase des jeweils aktuellen Pulszyklus ermittelt werden. Fällt jedoch dieses Maximum unter den vorgegebenen Grenzwert, so ist davon auszugehen, dass die Korrelation zwischen der vorgegebenen Testfunktion fester Frequenz und dem ersten Eingangssignal nicht mehr hinreichend hoch ist, und entsprechend eine andere Testfunktion auszuwählen ist.

In einer vorteilhaften Ausgestaltung wird im Normalmodus für eine Gruppe an Testfunktionen, deren Frequenzen einen Korridor um die Frequenz der ersten Testfunktion bilden, jeweils eine Korrelationsmessung mit dem ersten Eingangssignal durchgeführt, wobei anhand eines Maximums der besagten Korrelationsmessungen eine Aktualisierung der Frequenz der ersten Testfunktion erfolgt. Dies umfasst insbesondere, dass also im Normalmodus die erste Testfunktion (bzw. ihre Frequenz) zu einem ersten Zeitpunkt ermittelt wird, und daraufhin ein "Korridor" an weiteren Testfunktionen um die erste Testfunktion (also besagte Gruppe an Testfunktionen mit Frequenzen in einem Korridor, also insbesondere einem Intervall um die Frequenz der ersten Testfunktion) gewählt wird, welcher insbesondere eine echte Teilmenge aller verfügbaren Testfunktionen bildet, und für diese Testfunktionen jeweils die Korrelation mit dem ersten Eingangssignal ermittelt wird. Hierdurch kann bei langsamen Änderungen der Pulsfrequenz eine Abweichung leicht festgestellt werden, und eine neue erste Testfunktion aus dem genannten Korridor definiert werden. Der Korridor kann dann entsprechend verschoben werden.

Zweckmäßigerweise nimmt der erste Eingangswandler das erste Körperschallsignal im Gehörgang am Ohr der Person auf. Hierbei kann der erste Eingangswandler insbesondere für eine aktive Okklusionsunterdrückung vorgesehen und eingerichtet sein, welche im Hörinstrument für eine Kompensierung von zu lautem Körperschall im Gehörgang verwendet wird.

In einer Weiterbildung nimmt ein elektroakustischer zweiter Eingangswandler eines zweiten Hörinstrumentes des Hörsystems ein zweites Körperschallsignal am anderen Ohr der Person auf, woraus das Hilfssignal als ein zweites Eingangssignal erzeugt wird. Bevorzugt wird in diesem Fall eine Korrelationsmessung des ersten und des zweiten Eingangssignals durchgeführt, und anhand dem hieraus resultierenden Wert der Korrelation die Pulsfrequenz ermittelt. Dies bedeutet insbesondere, dass im Normalmodus die Pulsfrequenz aus den aktuellen Werten des ersten Eingangssignals ermittelt wird, und lediglich im Spezialmodus die aktuellen Werte des zweiten Eingangssignals herangezogen werden. Eine Verwendung eines zweiten Eingangssignals bietet sich insbesondere für ein binaurales Hörsystem an, da ein solches zweites Eingangssignal aus dem kontramedialen Hörinstrument bezogen werden kann, wo es z.B. für eine aktive Okklusionsunterdrückung verwendet wird. Es kann nämlich davon ausgegangen werden, dass ein Verlust der Pulsfrequenz bei einer Messung an einem Ohr (etwa infolge eines zu schlechten SNR dort) unabhängig von der Messung am anderen Ohr verläuft, sodass die am anderen Ohr gemessene Pulsfrequenz weiterhin stabil und aussagekräftig sein sollte, und daher für die erstgenannte, instabile Messung als Referenz dienen kann.

**In** einer weiter vorteilhaften Ausgestaltung wird als erster Sensor ein PPG-Sensor verwendet, wobei anhand des durch den PPG-Sensor erzeugten Hilfssignals eine Pulsmessung durchgeführt wird, wobei als erste Information eine Referenz-Pulsfrequenz der Person ermittelt wird, und daraufhin diese erste Information als Referenz für die nachfolgende Ermittlung der Pulsfrequenz anhand des ersten Eingangssignals, insbesondere nur anhand des ersten Eingangssignals, herangezogen wird. Bevorzugt wird dabei die so ermittelte Referenz-Pulsfrequenz als Referenz für die Korrelationsmessung herangezogen, wobei nach dem Ermitteln der Referenz-Pulsfrequenz als erster Information die Korrelationsmessung des ersten Eingangssignals insbesondere ohne aktuelle Signalanteile des Hilfssignals erfolgt.

**In** einer vorteilhaften Ausgestaltung wird als erster Sensor ein Beschleunigungssensor, welcher an einer Kopf- und/oder Halsschlagader der Person angeordnet ist, und/oder ein Elektrokardiogramm-Sensor verwendet (in diesem Fall wird als Hilfssignal ein Elektrokardiogramm der Person erstellt), wobei als erste Information anhand einer vom Beschleunigungssensor aufgezeichneten Bewegung der Kopf- bzw. Halsschlagader bzw. aus dem Elektrokardiogramm eine Referenz-Pulsfrequenz der Person ermittelt wird, und daraufhin diese erste Information als Referenz für eine nachfolgende Ermittlung der Pulsfrequenz anhand des ersten Eingangssignals, insbesondere nur anhand des ersten Eingangssignals, herangezogen wird. Die genannten Sensoren liefern ebenfalls einen Aufschluss über den Pulsschlag, und können zudem ebenfalls in einem Hörsystem Anwendung finden, sodass die nicht zusätzlich dediziert angeordnet werden müssen.

Insbesondere kann das Verfahren auch dazu verwendet werden, eine weitere bzw. andere kardiovaskuläre Größe zu bestimmen, also bspw. einen Blutdruck oder eine Differenz zwischen systolischem und diastolischem Blutdruck (sog. Blutdruck-Amplitude) eines Trägers des Hörinstruments. Gerade letztgenannter Wert als kardiovaskuläre Größe kann Aufschluss über mögliche Risiken von kardiovaskulären Erkrankungen liefern. Insbesondere kann hierfür eine Kontrastintensität herangezogen werden.

Nachfolgend wird ein Ausführungsbeispiel der Erfindung anhand von Zeichnungen näher erläutert. Hierbei zeigen jeweils schematisch:
- Fig. 1: in einem Blockschaltbild ein Hörgerät, welches für eine Pulsmessung seines Trägers eingerichtet ist, und
- Fig. 2: in einem Blockdiagramm den Ablauf einer Pulsmessung mittels des Hörgerätes nach Fig. 1.

Einander entsprechende Teile und Größen sind in allen Figuren jeweils mit denselben Bezugszeichen versehen.

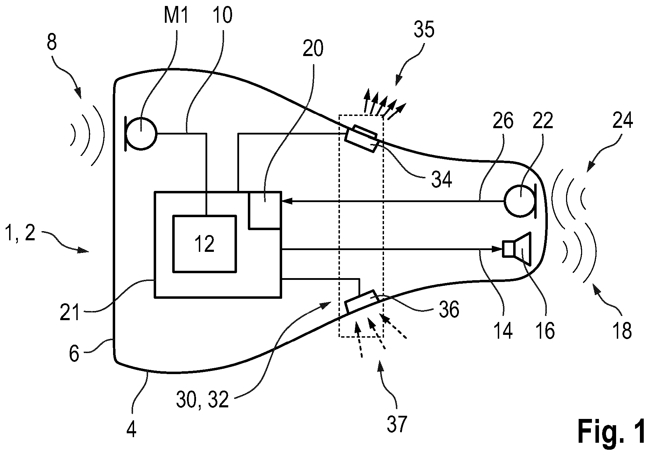

In Figur 1 ist schematisch in einem Blockschaltbild ein Hörinstrument 1 dargestellt, welches vorliegend als ein Hörgerät 2 (im engeren Sinne) ausgestaltet ist. Das Hörgerät 2 kann dabei einerseits monaural ausgestaltet sein (also als ein alleinstehendes Hörgerät gegeben sein), oder Teil eines binauralen Hörsystems mit einem weiteren Hörgerät (nicht dargestellt) vorliegen. Ebenso kann das Hörgerät 2 Teil eines nicht näher dargestellten Hörsystems sein, welches noch wenigstens ein mit dem Hörgerät 2 verbindbaren des Hilfsgerät (nicht dargestellt) wie zum Beispiel ein Fitness-Armband oder eine Smartwatch umfasst. Im in Figur 1 gezeigten Ausführungsbeispiel liegt das Hörgerät 2 als ein sogenanntes ITE-Gerät vor, die anhand von Figur 1 beschriebenen Merkmale und Funktionsweisen sind jedoch ohne weiteres auch auf ein BTE-Gerät, ein RIC-Gerät, ein CIC-Gerät oder andere denkbare Ausgestaltungsformen übertragbar.

Das Hörgerät 2 weist ein Gehäuse 4 mit einer Deckplatte 6 auf, welche bei einem bestimmungsgemäßen Tragen des Hörgerätes 2 in einem äußeren Gehörgang eines Trägers (nicht dargestellt) dem freien Raum neben dem Ohr des Trägers zugewandt ist. Im Bereich der Deckplatte 6 weist das Hörgerät 2 wenigstens ein äußeres Mikrofon M1 auf, welches dazu eingerichtet ist, ein Schallsignal 8 der Umgebung in ein Audiosignal 10 umzuwandeln. Im Bereich der Deckplatte 6 kann noch ein weiteres Mikrofon (nicht dargestellt) zur Erzeugung eines weiteren Audiosignals für eine direktionale Verarbeitung beider besagter Audiosignale angeordnet sein. Das Audiosignal 10 wird an eine Signalverarbeitungseinrichtung 12 des Hörgerätes 2 weitergeleitet, und dort gemäß den audiologischen Anforderungen des Trägers des Hörgerätes 2 verarbeitet, und hierbei insbesondere frequenzbandweise verstärkt und/oder komprimiert. Ebenso kann hierbei eine Anhebung von Sprachanteilen im Audiosignal 10 erfolgen, sowie eine (gegebenenfalls direktionale) Rauschunterdrückung.

Durch die wie beschrieben erfolgende Verarbeitung des Audiosignals 10 in der Signalverarbeitungseinrichtung 12 wird ein Ausgangssignal 14 erzeugt, welches durch einen Lautsprecher 16 des Hörgerätes 2 in ein Ausgangsschallsignal 18 umgewandelt wird. Das Ausgangsschallsignal 18, welches bevorzugt die für den Träger des Hörgerätes 2 benutzerspezifisch aufbereitete Entsprechung des Schallsignals 8 beinhaltet, wird in bestimmungsgemäßen Betrieb des Hörgerätes 2 in den Gehörgang des besagten Trägers ausgegeben, welcher beim Tragen wenigstens teilweise und üblicherweise nahezu vollständig durch das Gehäuse 4 verschlossen wird. Durch besagten Verschluss des Gehörgangs (nicht dargestellt) des Trägers entstehen hierbei sogenannte Okklusionseffekte, d. h., Körperschall, welcher durch die Haut an den Gehörgang ausgegeben wird, kann infolge des besagten Verschlusses desselben nicht entweichen, sondern wird stattdessen in erheblichem Umfang vom Trommelfell des Trägers des Hörgerätes 2 wahrgenommen.

Da hierbei beispielsweise beim Gehen oder auch bei einem bewegendes Kiefers dumpfe Geräusche entstehen können, welche durch den Träger als störend bis unangenehm empfunden werden können, weist das Hörgerät 2 ein Modul zur aktiven Okklusionseffekte-Unterdrückung (active occlusion cancellation, AOC) 20 auf, welches auf einer Steuereinheit 21 implementiert ist, auf welcher auch die Signalverarbeitungseinrichtung 12 umgesetzt ist. Für die AOC 20 ist zudem im Bereich des Lautsprechers 16 ein erster Eingangswandler 22 angeordnet, welcher vorliegend als ein Mikrofon ausgestaltet ist, und welcher dazu eingerichtet ist, im bestimmungsgemäßen Betrieb des Hörgerätes 2 ein erstes Körperschallsignal 24, welches im Gehörgang des Trägers emittiert wird, aufzunehmen und in ein erstes Eingangssignal 26 umzuwandeln. Anhand des ersten Eingangssignals 26 können dann die besagten Okklusionseffekte behoben werden, indem durch die AOC 20 dem Ausgangssignal 14 ein entsprechendes Kompensationssignal beigemischt wird.

Anhand des ersten Eingangswandlers 22 ist es jedoch auch möglich, eine Pulsmessung in noch zu beschreibender Weise durchzuführen. Hierfür wird u.a. das erste Eingangssignal 26 herangezogen, welches das erste Körperschallsignal 24 beinhaltet. Im ersten Körperschallsignal 24 sind zumeist auch Schallanteile eines Pulsschlags der Adern enthalten, welche den Gehörgang umgeben. Anhand dieser Schallanteile kann eine Pulsfrequenz gemessen werden, indem z.B. Periodizitäten im ersten Eingangssignal 26 identifiziert werden. Da die besagten Schallanteile des Pulsschlags im ersten Körperschallsignal 24 jedoch von vielen anderen Geräuschen überlagert werden, und somit bisweilen ein ungünstiges SNR vorliegen kann, weist das Hörgerät 2 zur Unterstützung der besagten Pulsmessung weiter einen ersten Sensor 30 auf, welcher vorliegend als ein PPG-Sensor 32 gegeben ist.

Dieser PPG-Sensor 32 umfasst wenigstens eine als LED ausgestaltete Lichtquelle 34 sowie einen als Photodiode ausgestalteten Lichtsensor 36, welcher entgegengesetzt zur Lichtquelle 34 angeordnet ist. Die Lichtquelle 34 und der Lichtsensor 36 sind hierbei mit der Steuereinheit 21 verbunden, welche einerseits die Ausgabe von ausgehenden Lichtsignalen 35 in das Gewebe des Gehörgangs durch die Lichtquelle 34 ansteuert, und andererseits die durch den Lichtsensor 36 im Gehörgang registrierten eingehenden Lichtsignale 37 analysiert. Die in die Haut und somit in das Gewebe des Gehörgangs ausgehenden Lichtsignale 35 propagieren dabei teilweise durch das Gewebe und auch durch die Gefäße im Gehörgang, und erfahren dabei eine mit dem Pulsschlag pulsierende Modulation. Anschließend treten Teile der so modulierten ausgehenden Lichtsignalen 35 seitlich wieder durch die Haut in den Gehörgang aus, und werden als die eingehenden Lichtsignale 37 vom Lichtsensor 36 registriert. Anhand der Modulationen der registrierten Lichtsignale 37 lässt sich nun ebenfalls eine Pulsfrequenz bestimmen. Diese Bestimmung mittels des PPG-Sensors weist jedoch infolge der ausgehenden Lichtsignale 35 einen höheren Energieverbrauch auf, als die oben genannte Pulsmessung anhand des ersten Eingangssignals 26.

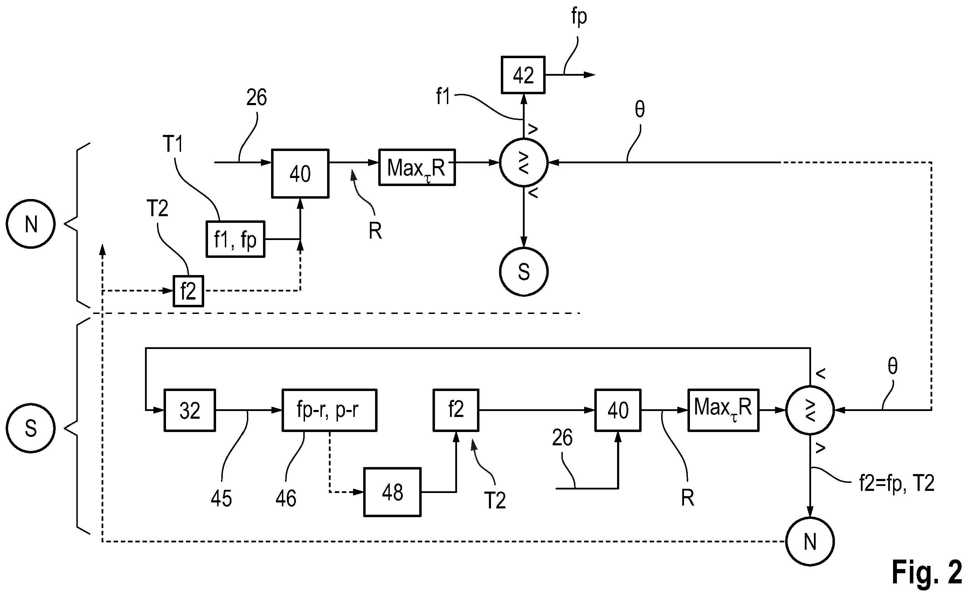

In Figur 2 ist daher in einem Blockdiagramm der Ablauf einer energieeffizienten und zugleich zuverlässigen Pulsmessung mittels des Hörgerätes nach Figur 1 dargestellt. In einem Normalmodus N wird dabei für das erste Eingangssignal 26, welches wie beschrieben durch den ersten Eingangswandler 22 aus dem Körperschallsignal 24 erzeugt wird, und Schallanteile eines Pulsschlags beinhaltet, eine Korrelationsmessung 40 bezüglich einer Testfunktion T1 (eines sog. Korrelators) durchgeführt, welcher eine Frequenz f1 und eine Phase p1 zugewiesen sind (die Frequenz f1 kann z.B. durch eine Periodizität der Testfunktion T1 gegeben sein, oder anhand einer inversen Dauer der Testfunktion T1 in der Zeit-Domäne). Die Auswahl der Testfunktion T1 wird im Folgenden noch beschrieben. Es wird also z.B. die Kreuzkorrelation R des ersten Eingangssignals 22 und der Testfunktion T1 berechnet, und dabei das Maximum Max_{τ} R hinsichtlich des Zeitargumentes τ der Kreuzkorrelation R gebildet. Liegt das besagte Maximum Max_{τ} R der Kreuzkorrelation über einem vorgegebenen Grenzwert θ, also Max_{τ} R > θ, so wird die Korrelation zwischen der Testfunktion T1 und dem ersten Eingangssignal 22 für hinreichend befunden. In diesem Fall wird die der Testfunktion T1 zugewiesene Frequenz f1 als Pulsfrequenz fp einer Pulsmessung 42 ausgegeben. Die Phase p1 der Testfunktion T1 wird zudem als Phase des bei der Pulsmessung 42 gemessenen Pulsschlags angenommen.

Fällt jedoch das Maximum Max_{τ} R der Kreuzkorrelation unter den vorgegebenen Grenzwert θ, also Max_{τ} R < θ, so liegt keine hinreichende Korrelation zwischen der Testfunktion T1 und dem ersten Eingangssignal 22 vor. Die Pulsmessung 42 mittels der Korrelation mit der Testfunktion T1 ist dann im Normalmodus N gescheitert, und ein Spezialmodus S wird initiiert. Im Spezialmodus S wird durch den PPG-Sensor 32 als ersten Sensor 30 ein Hilfssignal 45 gemessen, welches gegeben ist durch das Messignal der eingehenden Lichtsignale 37. Aus diesem Hilfssignal 45 kann nun als eine erste Information 46 über den Pulsschlag der Person eine Referenz-Pulsfrequenz fp-r bestimmt werden. Zudem kann ggf. auch eine Referenz-Phase p-r ermittelt werden. Das Ermitteln der Referenz-Pulsfrequenz fpr anhand des durch den PPG-Sensor 32 erzeugten Hilfssignals 45 ist bereits weiter oben beschrieben.

Anhand der Referenz-Pulsfrequenz fp-r (und ggf. anhand der Referenz-Phase p-r) kann nun im Spezialmodus S aus einer Mehrzahl 48 an Testfunktionen eine neue Testfunktion T2 als neuen Korrelator ausgewählt werden, welcher die entsprechende Referenz-Pulsfrequenz fp-r zugewiesen ist (bzw. welcher eine Frequenz f2 zugewiesen ist, welche der Referenz-Pulsfrequenz fp-r am nächsten kommt).

Nun wird die Korrelationsmessung 40 des ersten Eingangssignals 26 bezüglich der neuen Testfunktion T2 durchgeführt, d.h., es wird wie oben beschrieben die zugehörige Kreuzkorrelation R gebildet. Liegt dabei das Maximum Max_{τ} R oberhalb des vorgegebenen Grenzwertes θ, so wird die Korrelation zwischen der neuen Testfunktion T2 und dem ersten Eingangssignal 26 als hinreichend hoch angesehen. Die der neuen Testfunktion T2 zugeordnete Frequenz f2 wird als Pulsfrequenz fp ausgegeben, und liefert nun somit das Resultat der Pulsmessung 42. Im weiteren Verlauf kann nun die Pulsmessung wieder im Normalmodus N erfolgen, wobei nun die eben im Spezialmodus S vorgegebene, neue Testfunktion T2 als Korrelator für das erste Eingangssignal 26 verwendet wird. Im Fall, dass das Maximum der Kreuzkorrelation Max_{τ} R zwischen dem ersten Eingangssignal 26 und der neuen Testfunktion T2 unterhalb des vorgegebenen Grenzwertes θ liegt, wird im Spezialmodus S das Erfassen des Hilfssignals 45 anhand des PPG-Sensors 32 und das anschließende Ermitteln einer Referenz-Pulsfrequenz sowie die zugehörige Auswahl einer weiteren Testfunktion aus der Mehrzahl 42 an gegebenen Testfunktionen wiederholt.

In der beschriebenen Weise kann im Spezialmodus S auch eine Initiierung der Pulsmessung 42 erfolgen, d.h., nach einem Betriebsstart o.ä. kann zunächst eine Auswahl der geeigneten Testfunktion im Spezialmodus S mittels des PPG-Sensors 32 durchgeführt werden, um dann für den weiteren Verlauf die Pulsmessung 42 im Normalmodus N lediglich mit dem ersten Eingangssignal 26 (und der zuvor im Spezialmodus S vorgegebenen Testfunktion) durchzuführen. Da der erste Eingangswandler 22 im Vergleich zum PPG-Sensor 32 eine deutlich geringere Leistungsaufnahme hat, und entsprechend nur eine vernachlässigbare Batterieleistung verbraucht, ist die Pulsmessung 42 im Normalmodus N wesentlich energiesparender. Insbesondere kann dann auch jedes Mal in den Spezialmodus S gewechselt werden, wenn eine Änderung der Pulsfrequenz (z.B. bei sportlicher Betätigung) dazu führt, dass die Korrelation zwischen dem ersten Eingangssignal 26 und der entsprechenden Testfunktion "verloren geht" (also unter den vorgegebenen Grenzwert θ fällt).

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Hörinstrument
- 2: Hörgerät
- 4: Gehäuse
- 6: Deckplatte
- 8: Schallsignal
- 10: Audiosignal
- 12: Signalverarbeitungseinrichtung
- 14: Ausgangssignal
- 16: Lautsprecher
- 18: Ausgangsschallsignal
- 20: AOC (aktive Okklusionsunterdrückung)
- 21: Steuereinheit
- 22: erster Eingangswandler
- 24: erstes Körperschallsignal
- 26: erstes Eingangssignal
- 30: erster Sensor
- 32: PPG-Sensor
- 34: Lichtquelle
- 35: ausgehende Lichtsignale
- 36: Lichtsensor
- 37: eingehende Lichtsignale
- 40: Korrelationsmessung
- 42: Pulsmessung
- 45: Hilfssignal
- 46: erste Information
- 48: Mehrzahl (an Testfunktionen)

- f1, f2: Frequenz (der Testfunktion bzw. der neuen Testfunktion)
- fp: Pulsfrequenz
- fp-r: Referenz-Pulsfrequenz
- Max_{τ} R: Maximum (der Kreuzkorrelation)
- M1: äußeres Mikrofon
- N: Normalmodus
- p1: Phase (der ersten Testfunktion)
- p-r: Referenz-Phase
- R: Kreuzkorrelation
- S: Spezialmodus
- T1, T2: Testfunktion

- θ: vorgegebener Grenzwert

## Patentansprüche

1. Verfahren zur Pulsmessung einer Person mittels eines Hörsystems, welches wenigstens ein erstes Hörinstrument (1) mit einem elektroakustischen ersten Eingangswandler (22) umfasst,
wobei durch den ersten Eingangswandler (22) ein erstes Körperschallsignal (24) an einem Ohr der Person aufgenommen wird, und hierdurch ein erstes Eingangssignal (26) erzeugt wird, und
wobei anhand eines Amplitudenverlaufes des ersten Eingangssignals (26) eine Pulsfrequenz (fp) ermittelt wird,
**dadurch gekennzeichnet,**
**dass** in einem Normalmodus (N) die Pulsfrequenz (fp) anhand des Amplitudenverlaufes des ersten Eingangssignals (26) ermittelt wird, dass im Fall eines Fehlers beim Ermitteln der Pulsfrequenz (fp) im Normalmodus (N) in einen Spezialmodus (S) gewechselt wird, und
**dass** im Spezialmodus (S) anhand eines Hilfssignals (45) und/oder anhand einer Korrelationsmessung eine erste Information (46) über einen Pulsschlag der Person bezogen wird, und die Pulsfrequenz (fp) anhand der ersten Information (46) ermittelt wird.

2. Verfahren nach Anspruch 1,
wobei die Pulsfrequenz (fp) anhand der ersten Information (46) ermittelt wird, und
wobei die erste Information (46) wenigstens zwei Extrema und/oder wenigstens zwei steigende oder fallende Flanken von einer Einhüllenden des ersten Eingangssignals (26) und/oder vor einer Betragsfunktion des ersten Eingangssignals (26) umfasst.

3. Verfahren nach Anspruch 1 oder Anspruch 2,
wobei die Pulsfrequenz (fp) anhand einer Korrelationsmessung (40) des ersten Eingangssignals (26) ermittelt wird.

4. Verfahren nach Anspruch 3,
wobei für die Korrelationsmessung (40) eine Korrelation des ersten Eingangssignals (26) mit einer Testfunktion (T1, T2) ermittelt wird, welcher eine vorgegebene Frequenz (f1, f2) zugeordnet ist.

5. Verfahren nach Anspruch 4,
wobei die Frequenz (f1, f2) der Testfunktion (T1, T2) anhand der ersten Information (46) vorgegeben wird.

6. Verfahren nach Anspruch 4 oder Anspruch 5,
wobei die Testfunktion (T1, T2) anhand einer Mehrzahl (48) an verschiedenen Testfunktionen mit unterschiedlicher Frequenz (f1, f2) und/oder Phase (p1) anhand der ersten Information (46) ausgewählt wird, oder wobei für eine Mehrzahl (48) an verschiedenen Testfunktionen mit unterschiedlicher Frequenz (f1, f2) jeweils eine Korrelation des ersten Eingangssignals (26) mit einer Testfunktion aus der Mehrzahl (48) ermittelt wird, und die erste Testfunktion (T1, T2) aus der Mehrzahl (48) anhand eines Maximums der besagten Korrelationen ermittelt wird.

7. Verfahren nach einem der vorhergehenden Ansprüche wobei wenigstens zeitweise, insbesondere im Spezialmodus (S)
- durch einen ersten Sensor (30) des Hörsystems und/oder einen zweiten Eingangswandler des Hörsystems das Hilfssignal (45) erzeugt wird,
- anhand des Hilfssignals (45) die erste Information (46) über einen Pulsschlag der Person bezogen wird, und
- daraufhin anhand der ersten Information (46) und anhand des ersten Eingangssignals (26) die Pulsfrequenz (fp) ermittelt wird.

8. Verfahren nach Anspruch 6,
wobei im Spezialmodus (S) anhand des Maximums der besagten Korrelationen als erste Information die erste Testfunktion (T1, T2) aus der Mehrzahl (48) an Testfunktionen und/oder die Frequenz (f1, f2) der ersten Testfunktion (T1, T2) ermittelt wird.

9. Verfahren nach einem der vorhergehenden Ansprüche,
wobei nach einem erfolgreichen Ermitteln der Pulsfrequenz (fp) im Spezialmodus (S) wieder in den Normalmodus (N) gewechselt wird.

10. Verfahren nach Anspruch 9,
wobei nach dem Wechsel vom Spezialmodus (S) in den Normalmodus (N) die Pulsfrequenz (fp) anhand des ersten Eingangssignals (26) und der im vorigen Spezialmodus (S) bezogenen ersten Information (46) ermittelt wird.

11. Verfahren nach einem der vorhergehenden Ansprüche,
wobei im Normalmodus (N) für eine Gruppe an Testfunktionen, deren Frequenzen einen Korridor um die Frequenz (f1, f2) der ersten Testfunktion (T1, T2) bilden, jeweils eine Korrelationsmessung (40) mit dem ersten Eingangssignal (26) durchgeführt wird, und anhand eines Maximums der besagten Korrelationsmessungen (40) eine Aktualisierung der Frequenz (f1, f2) der ersten Testfunktion (T1, T2) erfolgt.

12. Verfahren nach einem der Ansprüche 3 bis 11,
wobei die erste Information (46) bezogen wird, wenn der Wert der Korrelationsmessung (40) unter einen vorgegebenen Grenzwert (θ) fällt.

13. Verfahren nach einem der vorhergehenden Ansprüche,
wobei der erste Eingangswandler (22) das erste Körperschallsignal (24) im Gehörgang am Ohr der Person aufnimmt, und/oder
wobei ein elektroakustischer zweiter Eingangswandler eines zweiten Hörinstrumentes des Hörsystems ein zweites Körperschallsignal am anderen Ohr der Person aufnimmt, und hieraus das Hilfssignal (46) als ein zweites Eingangssignal erzeugt wird.

14. Verfahren nach Anspruch 13,
wobei eine Korrelationsmessung (40) des ersten und des zweiten Eingangssignals durchgeführt wird, und anhand dem hieraus resultierenden Wert der Korrelation die Pulsfrequenz (fp) ermittelt wird.

15. Verfahren nach einem der Ansprüche 7 bis 14,
wobei
- als erster Sensor (30) ein Photoplethysmographie-Sensor (32) verwendet wird, und anhand des Hilfssignals (45) eine Pulsmessung durchgeführt wird, und/oder
als erster Sensor (30) ein Beschleunigungssensor verwendet wird, welcher an einer Kopf- und/oder Halsschlagader der Person angeordnet ist, und/oder
- als erster Sensor (30) ein Elektrokardiogramm-Sensor verwendet wird und dabei als Hilfssignal (45) ein Elektrokardiogramm der Person erstellt wird,
wobei als erste Information (46) anhand der Pulsmessung bzw. einer vom Beschleunigungssensor aufgezeichneten Bewegung der Kopf- bzw. Halsschlagader bzw. aus dem Elektrokardiogramm eine Referenz-Pulsfrequenz (fp-r) der Person ermittelt wird,
und wobei daraufhin diese erste Information (46) als Referenz für die nachfolgende Ermittlung der Pulsfrequenz (fp) anhand des ersten Eingangssignals (26), insbesondere nur anhand des ersten Eingangssignals (26), herangezogen wird.

16. Hörsystem, umfassend wenigstens ein erstes Hörinstrument (1) mit einem elektroakustischen ersten Eingangswandler (22), und eine Steuereinheit (21),
wobei das Hörsystem dazu eingerichtet ist, das Verfahren zur Pulsmessung einer Person nach einem der vorhergehenden Ansprüche durchzuführen, wenn das erste Hörinstrument (1) an einem Ohr der Person getragen wird.

## Claims

1. Method for measuring the pulse of a person by means of a hearing system, which comprises at least a first hearing instrument (1) with an electroacoustic first input transducer (22),
with a first structure-borne sound signal (24) being picked up by the first input transducer (22) at an ear of the person, and a first input signal (26) being generated as a result, and
wherein a pulse rate (fp) is determined on the basis of an amplitude profile of the first input signal (26), **characterized in that**,
in a normal mode (N), the pulse rate (fp) is determined on the basis of the amplitude profile of the first input signal (26),
**in that**, in the event of an error when determining the pulse rate (fp) in the normal mode (N), a change is made to a special mode (S), and
**in that**, in the special mode (S), a first item of information (46) about a pulse beat of the person is obtained on the basis of an auxiliary signal (45) and/or on the basis of a correlation measurement, and the pulse rate (fp) is determined on the basis of the first item of information (46).

2. Method according to Claim 1,
wherein the pulse rate (fp) is determined on the basis of the first item of information (46), and
wherein the first item of information (46) comprises at least two extremes and/or at least two rising or falling flanks of an envelope of the first input signal (26) and/or before an absolute value function of the first input signal (26).

3. Method according to Claim 1 or Claim 2,
wherein the pulse rate (fp) is determined on the basis of a correlation measurement (40) of the first input signal (26).

4. Method according to Claim 3,
wherein a correlation of the first input signal (26) is determined for the correlation measurement (40) by a test function (T1, T2), which is assigned a predetermined frequency (f1, f2).

5. Method according to Claim 4,
wherein the frequency (f1, f2) of the test function (T1, T2) is predetermined on the basis of the first item of information (46).

6. Method according to Claim 4 or Claim 5,
wherein the test function (T1, T2) is selected on the basis of a plurality (48) of different test functions with different frequencies (f1, f2) and/or phases (p1) on the basis of the first item of information (46), or
wherein, for a plurality (48) of different test functions with different frequencies (f1, f2), a correlation of the first input signal (26) with a test function is respectively determined from the plurality (48), and the first test function (T1, T2) is determined from the plurality (48) on the basis of a maximum of said correlations.

7. Method according to one of the preceding claims,
wherein, at least for a time, in particular in the special mode (S),
- the auxiliary signal (45) is generated by a first sensor (30) of the hearing system and/or a second input transducer of the hearing system,
- the first item of information (46) about a pulse beat of the person is obtained on the basis of the auxiliary signal (45), and
- then the pulse rate (fp) is determined on the basis of the first item of information (46) and on the basis of the first input signal (26).

8. Method according to Claim 6,
wherein, in the special mode (S), the first test function (T1, T2) is determined from the plurality (48) of test functions and/or the frequency (f1, f2) of the first test function (T1, T2) is determined on the basis of the maximum of said correlations as the first item of information.

9. Method according to one of the preceding claims,
wherein, after a successful determination of the pulse rate (fp) in the special mode (S), a change is made back to the normal mode (N).

10. Method according to Claim 9,
wherein, after the change from the special mode (S) to the normal mode (N), the pulse rate (fp) is determined on the basis of the first input signal (26) and the first item of information (46) obtained in the previous special mode (S).

11. Method according to one of the preceding claims,
wherein, in the normal mode (N), a correlation measurement (40) with the first input signal (26) is respectively carried out for a group of test functions of which the frequencies form a corridor around the frequency (f1, f2) of the first test function (T1, T2), and an updating of the frequency (f1, f2) of the first test function (T1, T2) takes place on the basis of a maximum of said correlation measurements (40).

12. Method according to one of Claims 3 to 11,
wherein the first item of information (46) is obtained when the value of the correlation measurement (40) falls below a predetermined limit value (θ).

13. Method according to one of the preceding claims,
wherein the first input transducer (22) picks up the first structure-borne sound signal (24) in the auditory canal at the ear of the person, and/or
wherein an electroacoustic second input transducer of a second hearing instrument of the hearing system picks up a second structure-borne sound signal at the other ear of the person, and from this the auxiliary signal (46) is generated as a second input signal.

14. Method according to Claim 13,
wherein a correlation measurement (40) of the first and second input signals is carried out, and the pulse rate (fp) is determined on the basis of the value of the correlation resulting from this.

15. Method according to one of Claims 7 to 14,
wherein
- a photoplethysmography sensor (32) is used as the first sensor (30), and a pulse measurement is carried out on the basis of the auxiliary signal (45), and/or an acceleration sensor arranged on a carotid artery of the person is used as the first sensor (30), and/or
- an electrocardiogram sensor is used as the first sensor (30) and in this case an electrocardiogram of the person is created as the auxiliary signal (45),
wherein a reference pulse rate (fp-r) of the person is determined as a first item of information (46) on the basis of the pulse measurement or a movement of the carotid artery recorded by the acceleration sensor or from the electrocardiogram,
and wherein this first item of information (46) is then used as a reference for the subsequent determination of the pulse rate (fp) on the basis of the first input signal (26), in particular only on the basis of the first input signal (26).

16. Hearing system, comprising at least a first hearing instrument (1) with an electroacoustic first input transducer (22), and a control unit (21),
wherein the hearing system is set up to carry out the method for measuring the pulse of a person as claimed in one of the preceding claims when the first hearing instrument (1) is worn at an ear of the person.

## Revendications

1. Procédé de mesure de pouls d'une personne au moyen d'un système auditif, qui comprend au moins un premier instrument auditif (1) doté d'un premier transducteur d'entrée électroacoustique (22),
dans lequel un premier signal de bruit corporel (24) est capté par le premier transducteur d'entrée (22) au niveau d'une oreille de la personne, et un premier signal d'entrée (26) est ainsi généré, et
dans lequel une fréquence de pouls (fp) est déterminée sur la base d'une courbe d'amplitude du premier signal d'entrée (26),
caractérisé en ce
dans un mode normal (N), la fréquence de pouls (fp) est déterminée sur la base de la courbe d'amplitude du premier signal d'entrée (26), en ce qu'en cas d'erreur lors de la détermination de la fréquence de pouls (fp) dans le mode normal (N), un basculement est effectué dans un mode spécial (S), et
en ce que, dans le mode spécial (S), une première information (46) concernant un battement de pouls de la personne est obtenue sur la base d'un signal auxiliaire (45) et/ou sur la base d'une mesure de corrélation, et la fréquence de pouls (fp) est déterminée sur la base de la première information (46).

2. Procédé selon la revendication 1,
dans lequel la fréquence de pouls (fp) est déterminée sur la base de la première information (46), et
dans lequel la première information (46) comprend au moins deux extrema et/ou au moins deux fronts montants ou descendants d'une enveloppe du premier signal d'entrée (26) et/ou avant une fonction de valeur absolue du premier signal d'entrée (26).

3. Procédé selon la revendication 1 ou la revendication 2,
dans lequel la fréquence de pouls (fp) est déterminée sur la base d'une mesure de corrélation (40) du premier signal d'entrée (26).

4. Procédé selon la revendication 3,
dans lequel, pour la mesure de corrélation (40), une corrélation du premier signal d'entrée (26) est déterminée au moyen d'une fonction de test (T1, T2) à laquelle est associée une fréquence prédéfinie (f1, f2).

5. Procédé selon la revendication 4,
dans lequel la fréquence (f1, f2) de la fonction de test (T1, T2) est prédéfinie sur la base de la première information (46).

6. Procédé selon la revendication 4 ou la revendication 5,
dans lequel la fonction de test (T1, T2) est sélectionnée sur la base de la première information (46) parmi une pluralité (48) de fonctions de test différentes ayant une fréquence (f1, f2) et/ou une phase (p1) différente, ou
dans lequel, pour une pluralité (48) de fonctions de test différentes ayant une fréquence (f1, f2) différente, une corrélation du premier signal d'entrée (26) avec une fonction de test issue de ladite pluralité (48) est respectivement déterminée, et la première fonction de test (T1, T2) est déterminée à partir de ladite pluralité (48) sur la base d'un maximum desdites corrélations.

7. Procédé selon l'une quelconque des revendications précédentes,
dans lequel, au moins temporairement, en particulier dans le mode spécial (S),
- le signal auxiliaire (45) est généré par un premier capteur (30) du système auditif et/ou un deuxième transducteur d'entrée du système auditif,
- la première information (46) concernant un battement de pouls de la personne est obtenue sur la base du signal auxiliaire (45), et
- la fréquence de pouls (fp) est ensuite déterminée sur la base de la première information (46) et sur la base du premier signal d'entrée (26).

8. Procédé selon la revendication 6,
dans lequel, dans le mode spécial (S), la première fonction de test (T1, T2) issue de la pluralité (48) de fonctions de test et/ou la fréquence (f1, f2) de la première fonction de test (T1, T2) est/sont déterminée(s) en tant que première information sur la base du maximum desdites corrélations.

9. Procédé selon l'une quelconque des revendications précédentes,
dans lequel, après une détermination réussie de la fréquence de pouls (fp) dans le mode spécial (S), un basculement est à nouveau effectué dans le mode normal (N).

10. Procédé selon la revendication 9,
dans lequel, après le basculement du mode spécial (S) vers le mode normal (N), la fréquence de pouls (fp) est déterminée sur la base du premier signal d'entrée (26) et de la première information (46) obtenue dans le mode spécial (S) précédent.

11. Procédé selon l'une quelconque des revendications précédentes,
dans lequel, dans le mode normal (N), pour un groupe de fonctions de test dont les fréquences forment un corridor autour de la fréquence (f1, f2) de la première fonction de test (T1, T2), une mesure de corrélation (40) avec le premier signal d'entrée (26) est respectivement effectuée, et une actualisation de la fréquence (f1, f2) de la première fonction de test (T1, T2) est effectuée sur la base d'un maximum desdites mesures de corrélation (40).

12. Procédé selon l'une quelconque des revendications 3 à 11,
dans lequel la première information (46) est obtenue lorsque la valeur de la mesure de corrélation (40) s'abaisse en dessous d'une valeur limite prédéfinie (θ).

13. Procédé selon l'une quelconque des revendications précédentes,
dans lequel le premier transducteur d'entrée (22) capte le premier signal de bruit corporel (24) dans le conduit auditif au niveau de l'oreille de la personne, et/ou
dans lequel un deuxième transducteur d'entrée électroacoustique d'un deuxième instrument auditif du système auditif capte un deuxième signal de bruit corporel au niveau de l'autre oreille de la personne, et le signal auxiliaire (46) est généré à partir de celui-ci en tant que deuxième signal d'entrée.

14. Procédé selon la revendication 13,
dans lequel une mesure de corrélation (40) du premier et du deuxième signal d'entrée est effectuée, et la fréquence de pouls (fp) est déterminée sur la base de la valeur de corrélation qui en résulte.

15. Procédé selon l'une quelconque des revendications 7 à 14,
dans lequel
- un capteur de photopléthysmographie (32) est utilisé en tant que premier capteur (30), et une mesure de pouls est effectuée sur la base du signal auxiliaire (45),
et/ou
un capteur d'accélération est utilisé en tant que premier capteur (30), et est disposé au niveau d'une artère carotide et/ou d'une artère temporale de la personne,
et/ou
- un capteur d'électrocardiogramme est utilisé en tant que premier capteur (30) et un électrocardiogramme de la personne est ainsi établi en tant que signal auxiliaire (45),
dans lequel une fréquence de pouls de référence (fp-r) de la personne est déterminée en tant que première information (46) sur la base de la mesure de pouls ou d'un mouvement de l'artère temporale ou de l'artère carotide enregistré par le capteur d'accélération, ou à partir de l'électrocardiogramme,
et dans lequel, après cela, cette première information (46) est utilisée comme référence pour la détermination suivante de la fréquence de pouls (fp) sur la base du premier signal d'entrée (26), en particulier uniquement sur la base du premier signal d'entrée (26).

16. Système auditif, comprenant au moins un premier instrument auditif (1) doté d'un premier transducteur d'entrée électroacoustique (22), et une unité de commande (21),
le système auditif étant configuré pour mettre en œuvre le procédé de mesure de pouls d'une personne selon l'une quelconque des revendications précédentes, lorsque le premier instrument auditif (1) est porté sur une oreille de la personne.
